(19) Europäisches Patentamt
European Patent Office
Office européen des brevets

(11) **EP 3 651 641 B1**

(12) **EUROPEAN PATENT SPECIFICATION**

(45) Date of publication and mention
of the grant of the patent:
**02.11.2022 Bulletin 2022/44**

(21) Application number: **18749144.4**

(22) Date of filing: **10.07.2018**

(51) International Patent Classification (IPC):
**A61B 5/0536** (2021.01)    **A61B 5/0538** (2021.01)
**A61B 5/00** (2006.01)

(52) Cooperative Patent Classification (CPC):
**A61B 5/0536; A61B 5/0538; A61B 5/444;**
A61B 2562/0209

(86) International application number:
**PCT/IB2018/055058**

(87) International publication number:
**WO 2019/012409 (17.01.2019 Gazette 2019/03)**

(54) **SYSTEM FOR MEASURING THE ELECTRICAL IMPEDANCE IN HUMAN TISSUES**

SYSTEM ZUR MESSUNG DER ELEKTRISCHEN IMPEDANZ IN MENSCHLICHEN GEWEBEN

SYSTÈME DE MESURE DE L'IMPÉDANCE ÉLECTRIQUE DANS DES TISSUS HUMAINS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **11.07.2017 IT 201700078157**

(43) Date of publication of application:
**20.05.2020 Bulletin 2020/21**

(73) Proprietor: **Politecnico di Milano**
**20133 Milano (IT)**

(72) Inventors:
 • **ALIVERTI, Andrea**
  **22100 Como (CO) (IT)**
 • **MERONI, Davide**
  **20851 Lissone (MB) (IT)**
 • **BOVIO, Dario**
  **28066 Galliate (NO) (IT)**
 • **CARPANO MAGLIOLI, Camilla**
  **13814 Pollone (BI) (IT)**
 • **MERONI, Emanuele**
  **6976 Lugano Castagnola (CH)**

(74) Representative: **Gatti, Enrico**
**Giambrocono & C. S.p.A.**
**Via E. Zambianchi, 3**
**24121 Bergamo (IT)**

(56) References cited:
**WO-A1-2008/095108     US-A1- 2013 211 280
US-A1- 2014 194 789**

 • **Orjan G Martinsen ET AL: "Invasive electrical
   impedance tomography for blood vessel
   detection", The open biomedical engineering
   journal, 1 January 2010 (2010-01-01), pages
   135-137, XP055463513, Netherlands Retrieved
   from the Internet:
   URL:https://www.ncbi.nlm.nih.gov/pmc/artic
   les/PMC3099542/pdf/TOBEJ-4-135.pdf [retrieved
   on 2018-03-28]**
 • **BROWN B H ET AL: "The Sheffield data collection
   system", CLINICAL PHYSICS AND
   PHYSIOLOGICAL MEASUREMENT, INSTITUTE
   OF PHYSICS PUBLISHING, BRISTOL, GB, vol. 8,
   no. 4A, 1 November 1987 (1987-11-01), pages
   91-97, XP020026373, ISSN: 0143-0815, DOI:
   10.1088/0143-0815/8/4A/012 cited in the
   application**

**Description**

The present invention relates to a system for measuring the electrical impedance in human tissues, more in particular to a system for measuring the electrical impedance for the electrical discrimination and characterization of human tissues and even more in particular for the discrimination of cancer tissues.

In particular, it regards a system for electrical-impedance tomography of human tissue and for the discrimination of various types of human tissues.

Cancer diagnosis is currently based upon radiological/endoscopic imaging and nuclear imaging, for morphological and functional evaluation of lesions, respectively. However, it is well known that differential diagnosis of lesions may at times be critical on account of the unsatisfactory sensitivity and of the specificity of both of the analysis methods and of histological examination of tissue samples.

In particular, it is frequently difficult to distinguish tumours from inflammatory masses. More invasive and costly procedures, such as image-guided biopsies, may also be inconclusive as tissue sampling may not be adequately targeted. A useful instrument for improving precision of current methods is represented by quantitative evaluation of the electrical properties of tissues.

In fact, the various types of tissue have different conductivity parameters, and, assuming that the presence of a neoplastic tissue alters this structure, the differences in the electrical properties of normal tissue and cancer tissue may constitute useful information in clinical applications. Moreover, by displaying the structural homogeneity and providing impedance maps to identify and analyse the area of interest more precisely may be useful in many diagnostic applications or during endoscopic operations, with the aim of studying suspect tissues.

Electrical-impedance tomography (EIT) is a technique of reconstruction of the electrical image of the tissues, which may provide a 2D cross-sectional view of the body of a patient. EIT, as compared to other techniques, is less expensive, smaller, and safer because it does not require the use of radiation, strong magnetic fields, or injection of radioactive markers.

Electrical-impedance spectroscopy (EIS) is a method for measuring electrical impedance of a substance as a function of the applied frequency.

The value of the impedance and the dependence of impedance upon frequency are both dependent upon tissue composition. Measurement of the impedance of tissues within a range of frequencies generates a spectrum that is characteristic of biological tissue. Modifications of the impedance spectrum may hence be directly correlated to the changes in the underlying nature of the tissue.

Document WO2008/095108 represents the closest prior art and discloses a system for measuring electrical impedance with a plurality of needles connected to a multiplexer.

The aim of the present invention is to provide a system for measuring electrical impedance in human tissues that can perform measurements with a high degree of accuracy and reliability.

Another aim is to provide a measurement system with an improved spatial resolution.

A further aim is to provide a measurement system that combines the EIT and EIS techniques.

According to the present invention, the above aims and others still are achieved by a system for measuring electrical impedance in human tissues according to claim 1.

Further characteristics of the invention are described in the dependent claims.

The advantages of the present solution over the solutions of the prior art are various.

The device developed is designed to explore the surface and the first layers of tissue. The central measurement core can be shifted to a lower level on the basis of the length of the needle.

The characteristics and advantages of the present invention will emerge clearly from the ensuing detailed description of a practical embodiment thereof, illustrated by way of nonlimiting example in the annexed plate of drawings, wherein:

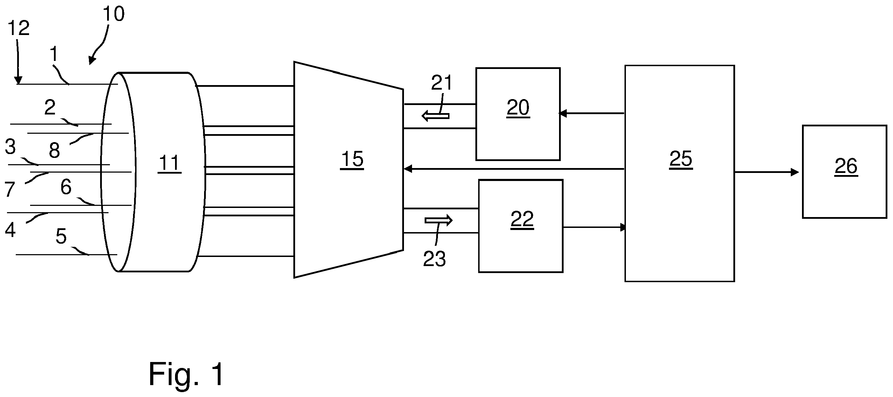

Figure 1 is a schematic illustration of a system for measuring electrical impedance in human tissues, according to the present invention.

With reference to the attached figure, a system for measuring electrical impedance in human tissues, according to the present invention, comprises eight electrodes 10 numbered 1 through 8. They are fixed in a plastic disk 11, having a thickness of approximately 0.5 cm, which keeps them parallel and all at the same height. The electrodes 10 pass through the disk 11, but are fixed thereto, and come out also at the back.

In the example of embodiment, eight electrodes are used, but a different number may be used, ranging, for example, between four and twelve.

The electrodes 10 are arranged along a circumference typically having a radius of 1.5 cm but in any case, preferably ranging between 0.5 and 5 cm.

The electrodes 10 are needles having a length of approximately 4 cm, of which approximately 2 cm exit from the disk 11 in the side defined as front and the remaining part of the needles in part is fixed in the disk 11 and in part exits from the back.

The needles have a diameter of approximately 0.3 mm and are made of stainless steel.

The length of the electrodes that comes out at the front from the disk 11 may vary according to the requirements and may be between 0.5 and 5 cm, according to the total length of the needle and the arrangement of the disk 11.

Each electrode 10 is entirely coated by an electrical insulator, for example polytetrafluoroethylene (PTFE), except for a portion at the tip 12 having a maximum length of 0.5 mm, typically 0.2 mm.

The electrodes 10 at the back are soldered to electric wires and are connected to one side of a multiplexer 15.

Connected on the other side of the multiplexer 15 is a current generator 20, which supplies a measurement current 21 in a selective way to pairs of electrodes 10.

The current generator 20 supplies an alternating current having a frequency ranging between 1 kHz and 1 MHz, more preferably between 10 kHz and 100 kHz.

The same measurement, applied to the same electrodes, is repeated for different frequencies in the aforesaid range so as to obtain a number of results at different frequencies.

Measurement of the impedance of the tissues varies as the frequency varies, and execution of a number of measurements (i.e., acquisition of a plurality of EIT images) enables a better evaluation of the alterations detected.

Connected again on the other side of the multiplexer 15 is a voltmeter 22, which reads the voltage 23 in a selective way from pairs of electrodes 10.

The current supplied is limited to 2 mA.

The multiplexer 15 may be physically made up of a number of multiplexers (for example, four) to be able to manage two inputs for the current generator and two outputs for the voltmeter and the eight electrodes.

The current generator 20, the voltmeter 22, and the multiplexer 15 are controlled by a control centre 25, typically a computer, a microcontroller, a tablet, or a smartphone, which are provided, *inter alia,* with a screen 26 for displaying the results of the measurements.

In the case where a portable device were to be provided, the communications between the various elements or between the computer and the device may be of a wireless type.

The control centre 25 is responsible for processing the data and reconstructing the impedance maps, for example, according to a protocol described in the paper by B. H. Brown and A. D. Seagar, "The Sheffield data collection system", Clin. Phys. Physiol. Meas., vol. 8, pp. 91-97, 1987.

This protocol uses a single-source EIT (Electrical Impedance Tomography) system with N electrodes in a 2D circular plane and is based upon a protocol that simultaneously activates two electrodes for current stimulation and two electrodes for voltage acquisition.

It requires the electrodes N to be evenly distributed in one and the same plane. The number of configurations for reconstructing the impedance map is computed as $N(N - 3)$. Considering eight electrodes, a total of forty configurations are managed by the algorithm, i.e., forty impedance measurements for each frequency.

An alternating current at a first frequency is injected between a pair of adjacent electrodes, for example 1 and 2, and the measurements are performed, in sequence, between pairs of adjacent electrodes that do not carry current, namely, V4-V3, V5-V4, V6-V5, V7-V6 and V8-V7. The measurement is repeated one or more times, changing the frequency.

Then an alternating current at the first frequency is injected between the electrodes 2 and 3, and the measurement between V5-V4, V6-V5, V7-V6, V8-V7, and V1-V8 is made. The measurement is repeated one or more times, changing the frequency.

The measurements are repeated on all the pairs of electrodes so as to supply current to all the pairs of electrodes and carry out the measurements between all the other electrodes present.

The control centre records the measurements made and supplies 2D images of the area measured, i.e., images having a size substantially equal to that of the circumference along which the electrodes 10 are arranged.

The measurements made are complex measurements defined by a real part and an imaginary part or else by magnitude and phase. Hence, it is possible to obtain images representing values for each type of elements of the complex number.

The images are obtained by assigning, for example, different colours to different impedance values, or else by representing graphically the variations of impedance.

In particular, the images are obtained as described in what follows.

The data are saved to a text file in which for each row the real part, the imaginary part, and the magnitude of the impedance at the various frequencies are stored. Hence, the text file is an NxM array in which each row contains the measurements at a different frequency. N is, for example, equal to 10 (from 1 kHz to 10 kHz), whereas M is, for example, equal to 3 (one column for the real values, one for the imaginary values, and one for the magnitude).

The control centre builds in real time a graph with the values of the third column that correspond to the values of the magnitude of the impedance.

The real part (column 1) and the imaginary part (column 2) are then plugged into the following equation to compute the phase at the various frequencies

$$\text{Phase(rads)} = \tan^{-1}\left(\frac{\text{I}}{\text{R}}\right)$$

During reconstruction of the EIT images, the data gathered are arranged in an array of, for example, 40x10 (i.e., 40 measurements of impedance for 10 different frequency values), in which each row is the frequency scan of a particular configuration of electrodes following the Sheffield protocol.

Preferably, prior to the measurements on the tissue, fifty measurements are recorded in physiological solution. From the data obtained, the median is computed. The EIT image will then be reconstructed in a differential way between the data recorded from the tissue and the median of the physiological solution calculated.

The measurement system described envisages a real-time dual analysis of the tissues. Real-time response is very important in the diagnostic field as it makes it possible to have immediately information on the nature of the suspect lesion and hence act accordingly with analyses that are more problem-oriented.

Via the EIS analysis, as soon as the measurement starts, it is possible to show on the screen of a PC a graph that is characteristic of the structure of the tissue. The graph will show the frequency response (10 Hz to 10 kHz) of the impedance. The various graphs are stored in memory as long as the operator deems it appropriate to be able to compare the profiles obtained by positioning the probe in different points of the tissue, whether cancer tissue or healthy tissue. It will consequently be possible to evaluate the differences thereof and identify the correct range of characteristic values of the cancer tissue. The control centre will indicate to the operator whether it is cancer tissue or other tissue. The range of characteristic values of the cancer tissue will be established via the continuous collection of data in order to be able to create a database for the various types of cancer.

Via EIS analysis, the control centre will display on the screen images at various frequencies, which identify the variation of impedance in the tissue. In particular, according to the type of tissue enclosed in the measurement range of the probe (within the electrodes arranged in a circle), it will colour the cancer tissue and the healthy tissue or the tissue of some other nature differently.

Positioning of the electrodes is made by arranging them on the surface of the human tissue and/or inserting them into the tissue to the desired depth allowed by the length of the electrodes and their positioning in the disk 11.

It is possible to obtain images at a number of frequencies and at a number of depths thanks to the practically total insulation of the electrodes with just a few tenths of a millimetre exposed and conductive.

The present solution may be applied also in the case where four electrodes are used applying an alternating current, at at least two different frequencies in succession, to a pair of needles and measuring the impedance between a further two needles.

From experiments it emerges that it is possible to distinguish cancer tissues from healthy tissues, on the basis of the images obtained with the present invention, where significant variations of the impedance measured are found and in particular where reduction of impedance is found.

A possible application of the present invention is also that of coupling the system to a biopsy needle in order to improve sampling of the tissue, for example by positioning the biopsy needle at the centre of the electrodes.

Standard biopsy procedure is subject to certain problems due to current endoscopic and/or radiological procedures, which do not enable correct targeting of the biopsy during tissue sampling, it being frequently difficult to identify the cancer tissue without collecting a considerable number of samples. Moreover, biopsy is a complex procedure and is associated with collateral problems for the patient, requires long hospitalization, and cannot guarantee real-time diagnosis of the illness.

Via the use of in depth EIT images it is hence possible to position the probe in the proximity of the suspect mass and reconstruct the electrical heterogeneity of the tissue. The maps will return a mapping of the tissue according to the different structure recorded, thus identifying and differentiating the cancer areas from those of another nature. Under the guide of the reconstructed electrical image, it will thus be possible to sample the tissue in a more focused way, reducing the amount of tissue sampled and consequently the number of false positives and false negatives, and also reducing the invasiveness of the procedure and harm to the patient.

**Claims**

1. A system for measuring electrical impedance in human tissues comprising a plurality of needles (10);

   said plurality of needles (10) being arranged along a circumference;
   where said plurality of needles (10) have a length greater than 0.5 cm;
   said plurality of needles (10) being coated with an electrically insulating material throughout their entire length except for a portion (12) having a maximum length of 0.5 mm; each of said plurality of needles (12) being connected to a multiplexer (15);
   an alternating-current generator (20) being connected to an input of said multiplexer (15);
   where said current generator (20) is configured to send a current (21) at at least two different frequencies ranging between 10 Hz and 1 MHz;
   a voltmeter (22) being connected to an output of said multiplexer (15);
   a control centre (25) connecting selectively and in succession said current generator (20) to first two needles of said plurality of needles (10);
   said control centre (25) being configured to control said multiplexer (15) so as to connect selectively and in succession said voltmeter (22) to second two needles of said plurality of needles (10) according to a predefined scheme;
   said control centre (25) being configured to measure the impedance between said second two needles at said at least two frequencies and repeat
   the measurements on all pairs of the needles;
   said control centre (25) being configured to calculate an image, having a size substantially equal to that of said circumference, for each of said at least two frequencies corresponding to said measurement of the impedance between said second two needles;

said control centre (25) being configured to supply said image to a system for displaying (26) said image;

wherein said plurality of needles (10) are N greater than 3 to be evenly distributed in one and the same plane and the number of measurements for reconstructing the image is computed as N(N - 3) for each frequency.

2. The system according to Claim 1, **characterized in that** said portion having a maximum length of 0.5 mm is located at the tip (12) of each needle of said plurality of needles (10).

3. The system according to Claim 1, **characterized in that** said current generator (20) is configured to send a current at at least two different frequencies ranging between 100 Hz and 100 kHz.

4. The system according to Claim 1, **characterized in that** said plurality of needles (10) range from 4 to 12 needles, and preferably 8 needles.

5. The system according to Claim 1, **characterized in that** said plurality of needles (10) have a length ranging between 0.5 and 5 cm, and preferably equal to 2.5 cm.

6. The system according to Claim 1, **characterized in that** said plurality of needles (20) are fixed to a disk (11).

7. The system according to Claim 6, **characterized in that** said plurality of needles (20) exit from both sides of said disk.

8. The system according to Claim 1, **characterized in that** said control centre (25) is configured to control said multiplexer so as to connect selectively and in succession said current generator (20) and said voltmeter (22) to said plurality of needles (10) in order to perform all the combinations of connection possible.

9. The system according to Claim 1, **characterized in that** it comprises a biopsy needle fixed on said disk (11).

**Patentansprüche**

1. Ein System zur Messung der elektrischen Impedanz in menschlichen Geweben, welches eine Vielzahl von Nadeln (10) umfasst;

wobei die genannte Vielzahl von Nadeln (10) an einem entsprechenden Umfang entlang angeordnet ist;

wobei die genannte Vielzahl von Nadeln (10) jeweils eine Länge von über 0,5 cm aufweist;

wobei die genannte Vielzahl von Nadeln (10) mit einem elektrisch isolierenden Material beschichtet ist, und zwar über ihre gesamte Länge, mit Ausnahme eines Abschnitts (12), welcher eine Höchstlänge von 0,5 mm aufweist; wobei jede der genannten Vielzahl von Nadeln (10) mit einem entsprechenden Multiplexer (15) verbunden ist;

wobei ein Wechselstromgenerator (20) an einen Eingang des genannten Multiplexers (15) angeschlossen ist;

wobei der genannte Stromgenerator (20) konfiguriert ist, um Strom (21) mit mindestens jeweils zwei verschiedenen Frequenzen zwischen 10 Hz und 1 MHz abzugeben;

wobei ein Spannungsmesser (22) an einen Ausgang des genannten Multiplexers (15) angeschlossen ist;

wobei eine Steuerzentrale (25) jeweils selektiv und nacheinander den genannten Stromgenerator (20) mit den beiden ersten Nadeln der genannten Vielzahl von Nadeln (10) verbindet;

wobei die genannte Steuerzentrale (25) konfiguriert ist, um den genannten Multiplexer (15) zu steuern, so dass der genannte Spannungsmesser (22) jeweils selektiv und nacheinander mit den zweiten beiden Nadeln der genannten Vielzahl von Nadeln (10) gemäß einem vorbestimmten Schema verbunden wird;

wobei die genannte Steuerzentrale (25) konfiguriert ist, um die Impedanz zwischen den genannten zweiten beiden Nadeln mit den genannten mindestens zwei Frequenzen zu messen und die Messungen jeweils an allen Nadelpaaren zu wiederholen;

wobei die genannte Steuerzentrale (25) konfiguriert ist, um ein Bild zu berechnen, welches eine dem genannten Umfang im Wesentlichen entsprechende Größe aufweist, wobei jede der genannten mindestens zwei Frequenzen der genannten Messung der Impedanz zwischen den genannten zweiten beiden Nadeln entspricht;

wobei die genannte Steuerzentrale (25) konfiguriert ist, um das genannte Bild jeweils an ein System zur Anzeige (26) des genannten Bilds zu liefern;

wobei die genannte Vielzahl von Nadeln (10) N größer als 3 ist, um gleichmäßig auf ein und derselben Ebene verteilt werden zu können und die Anzahl der Messungen zur Rekonstruktion des Bilds als N(N - 3) für jede Frequenz berechnet wird.

**2.** Das System gemäß Anspruch 1, **dadurch gekennzeichnet, dass** der genannte Abschnitt, welcher eine Höchstlänge von 0,5 mm aufweist, an der Spitze (12) jeder Nadel der genannten Vielzahl von Nadeln (10) angeordnet ist.

**3.** Das System gemäß Anspruch 1, **dadurch gekennzeichnet, dass** der genannte Stromgenerator (20) konfiguriert ist, um einen Strom mit mindestens zwei verschiedenen Frequenzen abzugeben, die sich jeweils zwischen 100 Hz und 100 kHz bewegen.

**4.** Das System gemäß Anspruch 1, **dadurch gekennzeichnet, dass** die genannte Vielzahl von Nadeln (10) von 4 bis 12 Nadeln reicht und vorzugsweise 8 Nadeln aufweist.

**5.** Das System gemäß Anspruch 1, **dadurch gekennzeichnet, dass** die genannte Vielzahl von Nadeln (10) eine Länge aufweist, die zwischen 0,5 und 5 cm liegt und vorzugsweise 2,5 cm entspricht.

**6.** Das System gemäß Anspruch 1, **dadurch gekennzeichnet, dass** die genannte Vielzahl von Nadeln (10) an einer entsprechenden Scheibe (11) befestigt ist.

**7.** Das System gemäß Anspruch 6, **dadurch gekennzeichnet, dass** die genannte Vielzahl von Nadeln (10) jeweils von beiden Seiten der genannten Scheibe ausgeht.

**8.** Das System gemäß Anspruch 1, **dadurch gekennzeichnet, dass** die genannte Steuerzentrale (25) konfiguriert ist, um den genannten Multiplexer zu steuern, so dass der genannte Stromgenerator (20) und der genannte Spannungsmesser (22) mit der genannten Vielzahl von Nadeln (10) jeweils selektiv und nacheinander verbunden werden, um sämtliche mögliche Anschlusskombinationen zu vollführen.

**9.** Das System gemäß Anspruch 1, **dadurch gekennzeichnet, dass** es eine Biopsienadel umfasst, welche jeweils an der genannten Scheibe (11) befestigt ist.

**Revendications**

**1.** Système de mesure de l'impédance électrique dans des tissus humains comprenant une pluralité d'aiguilles (10);

ladite pluralité d'aiguilles (10) étant disposées le long d'une circonférence;
où ladite pluralité d'aiguilles (10) ont une longueur supérieure à 0,5 cm;
ladite pluralité d'aiguilles (10) étant enduites avec un matériau isolant électriquement sur toute leur longueur à l'exception d'une portion (12) ayant une longueur maximale de 0,5 mm ; chacune de ladite pluralité d'aiguilles (10) étant reliée à un multiplexeur (15);
un générateur de courant alternatif (20) étant relié à une entrée dudit multiplexeur (15);
où ledit générateur de courant (20) est configuré pour envoyer un courant (21) à au moins deux fréquences différentes comprises entre 10 Hz et 1 MHz;
un voltmètre (22) étant relié à une sortie dudit multiplexeur (15);
un centre de commande (25) reliant sélectivement et successivement ledit générateur de courant (20) aux deux premières aiguilles de ladite pluralité d'aiguilles (10);
ledit centre de commande (25) étant configuré pour commander ledit multiplexeur (15) de manière à relier sélectivement et successivement ledit voltmètre (22) aux deux secondes aiguilles de ladite pluralité d'aiguilles (10) selon un schéma prédéfini;
ledit centre de commande (25) étant configuré pour mesurer l'impédance entre lesdites deux secondes aiguilles auxdites au moins deux fréquences et répéter les mesures sur toutes les paires d'aiguilles;
ledit centre de commande (25) étant configuré pour calculer une image, ayant une taille sensiblement égale à celle de ladite circonférence, pour chacune desdites au moins deux fréquences correspondant à ladite mesure de l'impédance entre lesdites deux secondes aiguilles;
ledit centre de commande (25) étant configuré pour fournir ladite image à un système pour afficher (26) ladite image;
où ladite pluralité d'aiguilles (10) sont N supérieures à 3 pour être distribuées uniformément dans un seul et même plan et le nombre de mesures pour reconstruire l'image est calculé comme N(N - 3) pour chaque fréquence.

**2.** Système selon la revendication 1, **caractérisé en ce que** ladite portion ayant une longueur maximale de 0,5 mm est située à la pointe (12) de chaque aiguille de ladite pluralité d'aiguilles (10).

**3.** Système selon la revendication 1, **caractérisé en ce que** ledit générateur de courant (20) est configuré pour envoyer un courant à au moins deux fréquences différentes comprises entre 100 Hz et 100 kHz.

**4.** Système selon la revendication 1, **caractérisé en ce que** ladite pluralité d'aiguilles (10) sont comprises entre 4 et 12 aiguilles, et de préférence 8 aiguilles.

**5.** Système selon la revendication 1, **caractérisé en**

**ce que** ladite pluralité d'aiguilles (10) ont une longueur comprise entre 0,5 et 5 cm, et de préférence égale à 2,5 cm.

6. Système selon la revendication 1, **caractérisé en ce que** ladite pluralité d'aiguilles (10) sont fixées à un disque (11).

7. Système selon la revendication 6, **caractérisé en ce que** ladite pluralité d'aiguilles (10) sortent des deux côtés dudit disque.

8. Système selon la revendication 1, **caractérisé en ce que** ledit centre de commande (25) est configuré pour commander ledit multiplexeur de manière à relier sélectivement et successivement ledit générateur de courant (20) et ledit voltmètre (22) à ladite pluralité d'aiguilles (10) afin de réaliser toutes les combinaisons de liaison possibles.

9. Système selon la revendication 1, **caractérisé en ce qu'**il comprend une aiguille de biopsie fixée sur ledit disque (11).

EP 3 651 641 B1

Fig. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2008095108 A

**Non-patent literature cited in the description**

- **B. H. BROWN ; A. D. SEAGAR.** The Sheffield data collection system. *Clin. Phys. Physiol. Meas.,* 1987, vol. 8, 91-97